# EUROPEAN PATENT SPECIFICATION

(11) **EP 4 188 111 B1**
(45) Date of publication and mention of the grant of the patent: **02.07.2025**
(21) Application number: 21790629.6
(22) Date of filing: 31.08.2021
(51) Int. Cl.: A23K 20/105, A23K 20/158, A23K 50/40

(54) **PET FOOD COMPOSITIONS**
HAUSTIERFUTTERZUSAMMENSETZUNGEN
COMPOSITIONS ALIMENTAIRES POUR ANIMAUX DE COMPAGNIE

(30) Priority: 01.09.2020 US 202063073130 P
(43) Date of publication of application: 07.06.2023
(73) Proprietor: Hill's Pet Nutrition, Inc., Topeka, KS 66603 (US)
(72) Inventor: JEWELL, Dennis, Lawrence, Kansas 66049 (US); MORGAN, Laura, Saint George, Kansas 66535 (US); JACKSON, Matthew, Topeka, Kansas 66604 (US); PANICKAR, Kiran, Lawrence, Kansas 66049 (US)
(74) Representative: Wichmann, Hendrik
(86) International application number: PCT/US2021/048353
(87) International publication number: WO 2022/051248

(56) References cited:
- US-A1- 2020 239 941

## Description

### BACKGROUND

The well-being of domestic animals is closely related to their feeding. Correct feeding should result in a fit and healthy pet. To achieve correct feeding, one may utilize certain ingredients and concentrations of those ingredients which yield beneficial effects for the animal. Such beneficial effects may include maintenance of body weight, increasing lean mass and/or decreasing body fat.

It would therefore be desirable to provide a pet food composition which may affect one or more of maintenance of body weight, increasing lean mass and/or decreasing body fat.
Methods for identifying a companion animal susceptible to treatment that reduces the risk of stone formation and pet food compositions for reducing the risk of stone formation are described US 2020/0239941 A1.

### BRIEF SUMMARY

Applicants have discovered that utilization of certain ingredients within pet food provides for effective health benefits. In one aspect, the health benefits may be to increase the lean body mass of the animal. In another aspect, the health benefits may be to decrease body fat of the animal. The present invention provides pet food compositions as defined in the claims comprising betaine; carnitine; and a source of medium chain triglycerides. The pet food compositions may be used in a method for increasing the lean body mass of a canine, comprising feeding the animal the pet food composition.

Further areas of applicability of the present disclosure will become apparent from the detailed description provided hereinafter. It should be understood that the detailed description and specific examples are intended for purposes of illustration only and that present the invention pertains to the pet food compositions defined in the claims.

### DETAILED DESCRIPTION

For illustrative purposes, the principles of the present invention are described by referencing various exemplary embodiments thereof. It is to be understood that the invention is defined in the claims and is not limited in its application to the details of any particular embodiment shown.

As used herein and in the appended claims, the singular forms "a", "an", and "the" include plural references unless the context dictates otherwise. The singular form of any class of the ingredients refers not only to one chemical species within that class, but also to a mixture of those chemical species. The terms "a" (or "an"), "one or more" and "at least one" may be used interchangeably herein. The terms "comprising", "including", "containing", and "having" may be used interchangeably. The term "include" should be interpreted as "include, but are not limited to". The term "including" should be interpreted as "including, but are not limited to".

As used throughout, ranges are used as shorthand for describing each and every value that is within the range. Any value within the range can be selected as the terminus of the range.

Unless otherwise specified, all percentages and amounts expressed herein and elsewhere in the specification should be understood to refer to percentages by weight of the total composition. Reference to a molecule, or to molecules, being present at a "wt. %" refers to the amount of that molecule, or molecules, present in the composition based on the total weight of the composition.

According to the present application, use of the term "about" in conjunction with a numeral value refers to a value that may be +/- 5% of that numeral. As used herein, the term "substantially free" is intended to mean an amount less than about 5.0 weight %, less than 3.0 weight %, 1.0 wt.%; preferably less than about 0.5 wt.%, and more preferably less than about 0.25 wt.% of the composition.

Unless defined otherwise, all technical and scientific terms used herein have the same meanings as commonly understood by one of ordinary skill in the art to which this invention belongs.

The present disclosure is directed toward pet food compositions which are useful for the treatment of domestic pets. In certain embodiments, the pet is a dog. In other embodiments, the pet is a cat.

The present inventors have surprisingly and unexpectedly discovered that providing animals a pet food diet comprising betaine, carnitine and coconut oil provides for enhanced health benefit for the animal. Such enhanced health benefit may be exemplified by numerous aspects. In a first aspect, the enhanced health benefit is a synergistic effect for an increase in the pet's lean mass. In another aspect, the enhanced health benefit is a synergistic effect for a decrease in the pet's body fat.

In one aspect, the present disclosure therefore provides pet food compositions as defined in the claims comprising betaine, carnitine, and coconut oil. In certain embodiments, the pet food is in a dry form. In certain embodiments, the pet food is in a wet form.

The pet food composition comprises betaine. Betaine is a modified amino acid consisting of glycine with three methyl groups and serves as a methyl donor in several metabolic pathways. The betaine may be present at various amounts or concentrations. In one embodiment, betaine may be present in an amount of from about 0.01% to about 2%, based on the total weight of the pet food composition. For example, the betaine may be present in an amount of about 0.01 weight %, about 0.02 weight %, about 0.05 weight %, about 0.08 weight %, about 0.1 weight %, about 0.15 weight %, about 0.2 weight %, about 0.25 weight %, about 0.3 weight %, about 0.35 weight %, about 0.4 weight %, about 0.45 weight %, about 0.5 weight %, about 0.55 weight %, about 0.6 weight %, about 0.65 weight %, about 0.7 weight %, about 0.75 weight %, about 0.8 weight %, about 0.85 weight %, about 0.9 weight %, about 1 weight %, about 1.1 weight %, about 1.2 weight %, about 1.3 weight %, about 1.4 weight %, about 1.5 weight %, about 1.6 weight %, about 1.7 weight %, about 1.8 weight %, about 1.9 weight %, or about 2 weight %. In another example, the betaine may be present in an amount of from about 0.2% to about 1%, about 0.2% to about 0.8%, or about 0.4% to about 0.6%, based on the total weight of the pet food composition. In another embodiment, betaine may be present in an amount of from about 0.02% to about 1.8%, based on the total weight of the pet food composition. In a further embodiment, betaine may be present in an amount of from about 0.1% to about 1.5%, based on the total weight of the pet food composition. In another embodiment, betaine may be present in an amount of from about 0.03% to about 1.2%, about 0.03% to about 1%, about 0.07% to about 0.8%, about 0.1% to about 0.7%, or about 0.2% to about 0.7%, based on the total weight of the pet food composition. In another example, betaine may be present in an amount of from about 0.03 weight % to about 1 weight %, about 0.07 weight % to about 0.9 weight %, about 0.1 weight % to about 0.9 weight %, about 0.2 weight % to about 0.9 weight %, about 0.3 weight % to about 0.8 weight %, about 0.35 weight % to about 0.75 weight %, or about 0.35 weight % to about 0.7 weight %, based on the total weight of the pet food composition. In a further embodiment, betaine is present in an amount of about 0.03% to about 1%, about 0.2% to about 0.95%, or about 0.3% to about 0.85%, based on the dry weight of the pet food composition. In a typical implementation, the amount of betaine present in the pet food composition may be from about 0.2 wt. % to about 0.6 wt. %, more typically about 0.5 wt. %.

The pet food composition comprises carnitine. Carnitine is a quaternary ammonium compound involved in metabolism in most mammals, plants, and some bacteria. Carnitine has been shown to be essential for the transfer of long-chain fatty acids across the inner mitochondrial membrane for subsequent β-oxidation. The carnitine may be present at various amounts or concentrations. In one embodiment, carnitine may be present in an amount of from about 0.01% to about 1%, based on the total weight of the pet food composition. For example, carnitine may be present in an amount of about 0.01 weight %, about 0.015 weight %, about 0.02 weight %, about 0.025 weight %, about 0.03 weight %, about 0.035 weight %, about 0.04 weight %, about 0.045 weight %, about 0.05 weight %, about 0.055 weight %, about 0.06 weight %, about 0.065 weight %, about 0.07 weight %, about 0.075 weight %, about 0.08 weight %, about 0.09 weight %, about 0.095 weight %, about 0.1 weight %, about 0.15 weight %, about 0.2 weight %, about 0.25 weight %, about 0.3 weight %, about 0.35 weight %, about 0.4 weight %, about 0.45 weight %, about 0.5 weight %, about 0.6 weight %, about 0.7 weight %, about 0.8 weight %, about 0.9 weight %, or about 1 weight %. In another example, carnitine may be present in an amount of from about 0.01% to about 0.9%, 0.015% to about 0.85%, about 0.02% to about 0.8%, 0.025% to about 0.075%, or about 0.025% to about 0.06%, based on the total weight of the pet food composition. In another embodiment, carnitine may be present in an amount of from about 0.01% to about 0.09%, from about 0.015% to about 0.08%, from about 0.015% to about 0.07%, from about 0.015% to about 0.06%, or from about 0.015% to about 0.04%, based on the total weight of the pet food composition. In a further embodiment, carnitine may be present in an amount of from about 0.01% to about 0.5%, based on the total weight of the pet food composition. In another embodiment, carnitine may be present in an amount of from about 0.02% to about 0.06%, about 0.025% to about 0.055%, about 0.025% to about 0.06%, about 0.025% to about 0.055%, or about 0.25% to about 0.05%, based on the total weight of the pet food composition. In a further embodiment, carnitine is present in an amount of about 0.01% to about 0.6%, about 0.015% to about 0.055%, or about 0.015% to about 0.05%, based on the dry weight of the pet food composition. In a typical implementation, the amount of carnitine present in the pet food composition may be from about 0.01 wt. % to about 0.06 wt. %, more typically about 0.03 wt. %.

The pet food composition comprises a source of medium chain triglycerides such as, for example, coconut oil. The coconut oil may be present at various amounts or concentrations. In one embodiment, coconut oil may be present in an amount of from about 1% to about 5%, based on the total weight of the pet food composition. For example, coconut oil may be present in an amount of about 1 weight %, about 1.2 weight %, about 1.4 weight %, about 1.6 weight %, about 1.8 weight %, about 2 weight %, about 2.1 weight %, about 2.2 weight %, about 2.3 weight %, about 2.4 weight %, about 2.5 weight %, about 2.6 weight %, about 2.7 weight %, about 2.8 weight %, about 2.9 weight %, about 3 weight %, about 3.2 weight %, about 3.4 weight %, about 3.6 weight %, about 3.8 weight %, about 4 weight %, about 4.2 weight %, about 4.4 weight %, about 4.6 weight %, about 4.8 weight %, or about 5 weight %. In another example, coconut oil may be present in an amount of from about 1% to about 4%, 1.2% to about 3.8%, about 1.4% to about 3.6%, 1.6% to about 3.4%, about 1.8% to about 3.2%, about 2% to about 3%, based on the total weight of the pet food composition. In another embodiment, coconut oil may be present in an amount of from about 1% to about 3.5%, from about 1.2% to about 3.3%, from about 1.4% to about 3.1%, from about 1.5% to about 3%, or from about 1.6% to about 2.9%, based on the total weight of the pet food composition. In a further embodiment, coconut oil may be present in an amount of from about 0.05% to about 3.5%, based on the total weight of the pet food composition. In another embodiment, coconut oil may be present in an amount of from about 0.07% to about 3.5%, about 0.1% to about 3.5%, about 0.25% to about 3.5%, about 0.35% to about 3.5%, or about 0.5% to about 3.5%, based on the total weight of the pet food composition. In a further embodiment, coconut oil is present in an amount of about 1% to about 4%, about 1.5% to about 3.5%, or about 1.5% to about 3%, based on the dry weight of the pet food composition. In a typical implementation, the amount of coconut oil present in the pet food composition may be from about 1.5 wt. % to about 3.5 wt. %, more typically about 2.3 wt. %.

The weight ratio of betaine to carnitine is from 13:1 to 20:1. For example, the ratio of betaine to carnitine may be 13:1, about 14:1, about 15:1, about 16:1, about 17:1, about 18:1, about 19:1, or 20:1. In certain embodiments, the ratio of betaine to carnitine is from about 14:1 to about 19:1, or from about 15:1 to about 18:1, based on the total weight of the pet food composition. In further embodiments, the ratio of betaine to carnitine is about 16.66:1.

The weight ratio of betaine to the source of medium chain triglycerides (e.g., coconut oil) is from 1:3 to 1:7. For example, the ratio of betaine to coconut oil may be 1:3, about 1:3.2, about 1:3.4, about 1:3.6, about 1:3.8, about 1:4, about 1:4.2, about 1:4.4, about 1:4.6, about 1:4.8, about 1:5, about 1:5.2, about 1:5.4, about 1:5.6, about 1:5.8, about 1:6, about 1:6.5, or 1:7. In certain embodiments, the ratio of betaine to coconut oil is from 1:3 to about 1:6, or from about 1:3.5 to about 1:5.5, based on the total weight of the pet food composition. In further embodiments, the ratio of betaine to coconut oil is from about 1:3.3 to about 1:6, or from about 1:3.6 to about 1:5.2. In further embodiments, the ratio of betaine to coconut oil is about 1:4.6.

The mass ratio of carnitine to the coconut oil may vary as long as the weight ratios of betaine to carnitine and betaine to the source of medium chain triglycerides is within the ranges recited in claim 1. In certain embodiments, the ratio of carnitine to coconut oil is from about 1:50 to about 1:100, based on the total weight of the pet food composition. For example, the ratio of carnitine to coconut oil may be about 1:50, about 1:55, about 1:60, about 1:62, about 1:64, about 1:66, about 1:68, about 1:70, about 1:72, about 1:74, about 1:76, about 1:78, about 1:80, about 1:82, about 1:84, about 1:86, about 1:88, about 1:90, about 1:92, about 1:94, about 1:96, about 1:98, or about 1:100. In certain embodiments, the ratio of carnitine to coconut oil is from about 1:60 to about 1:90. In certain embodiments, the ratio of carnitine to coconut oil is from about 1:65 to about 1:85. In further embodiments, the ratio of carnitine to coconut oil is about 1:60 to about 1:95, from about 1:65 to about 1:90, or from about 1:65 to about 1:85, based on the total weight of the pet food composition. In further embodiments, the ratio of carnitine to coconut oil is from about 1:60 to about 1:90, from about 1:65 to about 1:85, or from about 1:70 to about 1:80. In further embodiments, the ratio of carnitine to coconut oil is about 1:76.66.

The combined total mass amount of the betaine, carnitine and/or coconut oil may vary. In certain embodiments, the betaine and the carnitine are present in an amount of from about 0.2% to about 1.5%, from about 0.3% to about 1.2%, or from about 0.4% to about 1%, based on the dry weight of the pet food composition. In certain embodiments, the betaine and the carnitine are present in an amount of from about 0.3% to about 1%, from about 0.4% to about 0.8%, or from about 0.4% to about 0.6%, based on the dry weight of the pet food composition.

In certain embodiments, the total amount of betaine and the coconut oil present in the composition is an amount of from about 0.5% to about 6%, from about 1% to about 5%, or from about 1.5% to about 4.5%, based on the dry weight of the pet food composition. In certain embodiments, the total amount of betaine and the coconut oil present in the composition is an amount of from about 1% to about 5%, from about 1.5% to about 4.5%, or from about 1.8% to about 3.8%, based on the dry weight of the pet food composition.

In certain embodiments, the total amount of carnitine and the coconut oil present in the composition is an amount of from about 0.5% to about 7%, from about 1% to about 6%, or from about 1% to about 4%, based on the dry weight of the pet food composition. In certain embodiments, the total amount of carnitine and the coconut oil present in the composition is an amount of from about 1% to about 5%, from about 1.5% to about 4%, or from about 1.5% to about 3.5%, based on the dry weight of the pet food composition.

In certain embodiments, the total amount of betaine, carnitine and coconut oil present in the composition is an amount of from about 0.6% to about 8%, from about 1% to about 7%, from about 1% to about 6%, or from about 1% to about 5%, based on the dry weight of the pet food composition. In certain embodiments, the total amount of betaine, carnitine and coconut oil present in the composition is an amount of from about 1% to about 5%, from about 1.5% to about 4%, or from about 1.8% to about 3.8%, based on the dry weight of the pet food composition.

The compositions of the present invention may optionally comprise additional ingredients suitable for use in pet food compositions. Examples of such ingredients include, but are not limited to, carbohydrates, dietary fibers, amino acids, minerals, trace elements, vitamins, additives.

Carbohydrates can be supplied by any of a variety of sources known by those skilled in the art, including oat fiber, cellulose, peanut hulls, beet pulp, parboiled rice, corn starch, corn gluten meal, and any combination of those sources. Grains supplying carbohydrates can include, but are not limited to, wheat, corn, barley, and rice. Carbohydrates content of foods can be determined by any number of methods known by those of skill in the art. Generally, carbohydrate percentage can be calculated as nitrogen free extract ("NFE"), which can be calculated as follows: NFE=100%-moisture %-protein %-fat %-ash %-crude fiber %.

Dietary fiber refers to components of a plant which are resistant to digestion by an animal's digestive enzymes. Dietary fiber includes soluble and insoluble fibers. Soluble fibers are resistant to digestion and absorption in the small intestine and undergo complete or partial fermentation in the large intestine, e.g., beet pulp, guar gum, chicory root, psyllium, pectin, blueberry, cranberry, squash, apples, oats, beans, citrus, barley, or peas. Insoluble fibers can be supplied by any of a variety of sources, including, for example, cellulose, whole wheat products, wheat oat, corn bran, flax seed, grapes, celery, green beans, cauliflower, potato skins, fruit skins, vegetable skins, peanut hulls, and soy fiber. Crude fiber includes indigestible components contained in cell walls and cell contents of plants such as grains, for example, hulls of grains such as rice, corn, and beans. Typical fiber amounts in compositions of the present disclosure can be from about 0 to 10%, or about 1% to about 5%.

Amino acids, including essential amino acids, can be added to the compositions of the present disclosure as free amino acids, or supplied by any number of sources, e.g., crude protein, to the compositions of the present disclosure. Essential amino acids are amino acids that cannot be synthesized de novo, or in sufficient quantities by an organism and thus must be supplied in the diet. Essential amino acids vary from species to species, depending upon the organism's metabolism. For example, it is generally understood that the essential amino acids for dogs and cats (and humans) are phenylalanine, leucine, methionine, lysine, isoleucine, valine, threonine, tryptophan, histidine and arginine. In addition, taurine, while technically not an amino acid but a derivative of cysteine, is an essential nutrient for cats.

The compositions of the present invention may optionally comprise fat. Fat can be supplied by any of a variety of sources known by those skilled in the art, including meat, meat by-products, fish oil, and plants. Plant fat sources include wheat, flaxseed, rye, barley, rice, sorghum, corn, oats, millet, wheat germ, corn germ, soybeans, peanuts, and cottonseed, as well as oils derived from these and other plant fat sources. The compositions of the present disclosure may contain at least about 9% (or from about 9% to about 35%, or from about 10% to about 25%, or from about 15% to about 22%) total fat.

In some embodiments, the present invention provides a pet food composition comprising an omega-6 polyunsaturated fatty acid supplement. In certain embodiments, the composition comprises one or more omega-3 polyunsaturated fatty acids. In some embodiments, the omega-6 polyunsaturated fatty acid supplement comprises arachidonic acid (AA). In some embodiments, the omega-3 polyunsaturated fatty acid comprises eicosapentaenoic acid (EPA) and/or docosahexaenoic acid (DHA). In some embodiments, the present invention provides a pet food composition comprising arachidonic acid (AA), eicosapentaenoic acid (EPA) and docosahexaenoic acid (DHA), in a ratio of AA:(EPA+DHA) from about 0.4:1 to about 0.6:1, and a ratio of total omega-6 fatty acids to total omega-3 fatty acids (n6:n3) ranges from about 4:1 to about 7:1.

In certain embodiments, the pet food composition may comprise alpha-linolenic acid (ALA). The alpha-linolenic acid may be present in an amount of from about 0.3% to about 1%, based on the dry weight of the pet food composition. For example, the amount of alpha-linolenic acid present in the pet food composition may be about 0.1 wt. %, about 0.2 wt. %, about 0.3 wt. %, about 0.4 wt. %, about 0.5 wt. %, about 0.6 wt. %, about 0.7 wt. %, about 0.8 wt. %, about 0.9 wt. %, or about 1 wt. %. In another embodiment, the amount of alpha-linolenic acid present in the pet food composition may be from about 0.1 wt. % to about 0.9 wt. %, about 0.1 wt. % to about 0.8 wt. %, about 0.1 wt. % to about 0.7 wt. %, about 0.1 wt. % to about 0.6 wt. %, or about 0.1 wt. % to about 0.5 wt. %. In a typical implementation, the amount of alpha-linolenic acid present in the pet food composition may be from about 0.2 wt. % to about 0.6 wt. %, more typically about 0.55 wt. %.

The compositions of the present disclosure can also contain one or more minerals and/or trace elements, e.g., calcium, phosphorus, sodium, potassium, magnesium, manganese, copper, zinc, chromium, molybdenum, selenium, or iron salts having counterions such as, for example chloride, iodide, fluoride, sulfide or oxide, in amounts required to avoid deficiency and maintain health. These amounts are known by those of skill in the art, for example, as provided in the Official Publication of the Associate of American Feed Control Officials, Inc. ("AAFCO"), Nutrient Requirements of Dogs and Cats, 2006. Typical mineral amounts are about 0.1% to about 4% or about 1% to about 2%.

The compositions of the present invention can also include vitamins in amounts required to avoid deficiency and maintain health. These amounts, and methods of measurement are known by those skilled in the art. For example, the Official Publication of the Associate of American Feed Control Officials, Inc. ("AAFCO"), Nutrient Requirements of Dogs and Cats, 2006 provides recommended amounts of such ingredients for dogs and cats. As contemplated herein, vitamins can include, but are not limited to, vitamin A, vitamin B.sub.1, vitamin B.sub.2, vitamin B.sub.6, vitamin B.sub.12, vitamin C, vitamin D, vitamin E, vitamin H (biotin), vitamin K, folic acid, choline, inositol, niacin, and pantothenic acid. Typical vitamin amounts in the composition of the invention are about from 0 to about 3% or about 1% to about 2%.

The compositions of the present disclosure can additionally comprise other additives such as palatability enhancers and stabilizers in amounts and combinations familiar to one of skill in the art. Stabilizing substances include, for example, substances that tend to increase the shelf life of the composition. Other examples of other such additives potentially suitable for inclusion in the compositions of the invention include, for example, preservatives, colorants, antioxidants, flavorants, synergists and sequestrants, packaging gases, stabilizers, emulsifiers, thickeners, gelling agents, and humectants. Examples of emulsifiers and/or thickening agents include, for example, gelatin, cellulose ethers, starch, starch esters, starch ethers, and modified starches. The concentration of such additives in the composition typically can be up to about 5% by weight. In some embodiments, the concentration of such additives (particularly where such additives are primarily nutritional balancing agents, such as vitamins and minerals) is from about 0% to about 2.0% by weight. In some embodiments, the concentration of such additives (again, particularly where such additives are primarily nutritional balancing agents) is from about 0% to about 1.0% by weight.

Foods of any consistency or moisture content are contemplated, e.g., the compositions of the present invention can be, for example, a dry, moist or semi-moist animal food composition. In some embodiments, the moisture content is from about 3% to about 90% of the total weight of the composition. "Semi-moist" refers to a food composition containing from about 25 to about 35% moisture. "Moist" food refers to a food composition that has a moisture content of about 60 to 90% or greater. "Dry" food refers to a food composition with about 3 to about 11% moisture content and is often manufactured in the form of small bits or kibbles.

In preparing a composition of the present invention in wet or canned form, any ingredient (e.g., betaine, carnitine, coconut oil) generally can, for example, be incorporated into the composition during the processing of the formulation, such as during and/or after mixing of other components of the composition. Distribution of these components into the composition can be accomplished by conventional means. For example, ground animal and poultry proteinaceous tissues are mixed with the other ingredients, including fish oils, cereal grains, other nutritionally balancing ingredients, special-purpose additives (e.g., vitamin and mineral mixtures, inorganic salts, cellulose and beet pulp, bulking agents); and water that is sufficient for processing is also added. These ingredients can be mixed in a vessel suitable for heating while blending the components. Heating of the mixture can be effected using any suitable manner, such as, for example, by direct steam injection or by using a vessel fitted with a heat exchanger. Following the addition of the last ingredient, the mixture can be heated to a temperature range of from about 50 °F (10 °C) to about 212 °F (100 °C). In some instances, the mixture can be heated to a temperature range of from about 70 °F. (21 °C) to about 140 °F (60 °C). Temperatures outside these ranges are generally acceptable but may be commercially impractical without use of other processing aids. When heated to the appropriate temperature, the material will typically be in the form of a thick liquid. The thick liquid can be filled into cans. When filled into cans, a lid is applied, and the container is hermetically sealed. The sealed can is then placed into conventional equipment designed to sterilize the contents. This is usually accomplished by heating to temperatures of greater than about 230 °F (110 °C) for an appropriate time, which is dependent on, for example, the temperature used and the composition.

Pet food compositions can alternatively be prepared in a dry form using conventional processes. Typically, dry ingredients, including, for example, animal protein, plant protein, grains, are ground and mixed together. Moist or liquid ingredients, including, for example, fats, oils, animal protein, water, are then added to and mixed with the dry mix. The mixture is then processed into kibbles or similar dry pieces. Kibble is often formed using an extrusion process in which the mixture of dry and wet ingredients is subjected to mechanical work at a high pressure and temperature, and forced through small openings and cut off into kibble by a rotating knife. The wet kibble is then dried and optionally coated with one or more topical coatings which may include, for example, flavors, fats, oils, powders. Kibble also can be made from the dough using a baking process, rather than extrusion, wherein the dough is placed into a mold before dry-heat processing.

Further, a method for increasing the lean body mass of a canine or feline is described herein, comprising feeding the animal a pet food composition comprising betaine, carnitine and coconut oil; in an amount effective to increase the lean body mass of the animal. Such increase in the lean body mass may be more than would occur under conditions where one or more of the specified ingredients are not present. The betaine may be present in an amount of about 0.03% to about 1%, based on the dry weight of the pet food composition. The carnitine may be present in an amount of about 0.01% to about 0.06%, based on the dry weight of the pet food composition. The coconut oil may be present in an amount of about 1.5% to about 3%, based on the dry weight of the pet food composition. The ratio of ratio of betaine to carnitine is from 13:1 to 20:1, e.g., from about 14:1 to about 19:1, or from about 15:1 to about 18:1. The ratio of betaine to coconut oil is from 1:3 to 1:7, e.g., from 1:3 to about 1:6, from about 1:3.3 to about 1:5.5, or from about 1:3.6 to about 1:5.2. The ratio of carnitine to coconut oil is from about 1:70 to about 1:90, from about 1:65 to about 1:85, or from about 1:70 to about 1:80. The betaine and the carnitine may be present in an amount of from about 0.3% to about 1%, from about 0.4% to about 0.8%, or from about 0.4% to about 0.6%, based on the dry weight of the pet food composition. The betaine and the coconut oil may be present in an amount of from about 1% to about 5%, from about 1.5% to about 4.5%, or from about 1.8% to about 3.8%, based on the dry weight of the pet food composition. The carnitine and the coconut oil may be present in an amount of from about 1% to about 5%, from about 1.5% to about 4%, or from about 1.5% to about 3.5%, based on the dry weight of the pet food composition. The betaine, carnitine and coconut oil may be present in an amount of from about 1% to about 5%, from about 1.5% to about 4%, or from about 1.8% to about 3.8%, based on the dry weight of the pet food composition.

Further, a method for decreasing the body fat of a canine or feline is described herein, comprising feeding the animal a pet food composition comprising betaine, carnitine, and coconut oil; in an amount effective to decrease the body fat of the animal. Such decrease in the body fat may be more than would occur under conditions where one or more of the specified ingredients (i.e. betaine, carnitine, coconut oil) are not present. The betaine may be present in an amount of about 0.03% to about 1%, based on the dry weight of the pet food composition. The carnitine may be present in an amount of about 0.01% to about 0.06%, based on the dry weight of the pet food composition. The coconut oil may be present in an amount of about 1.5% to about 3%, based on the dry weight of the pet food composition. The ratio of ratio of betaine to carnitine is from 13:1 to 20:1, e.g., from about 14:1 to about 19:1, or from about 15:1 to about 18:1. The ratio of betaine to coconut oil is from 1:3 to 1:7, e.g., from 1:3 to about 1:6, from about 1:3.3 to about 1:5.5, or from about 1:3.6 to about 1:5.2. The ratio of carnitine to coconut oil is from about 1:70 to about 1:90, from about 1:65 to about 1:85, or from about 1:70 to about 1:80. The betaine and the carnitine may be present in an amount of from about 0.3% to about 1%, from about 0.4% to about 0.8%, or from about 0.4% to about 0.6%, based on the dry weight of the pet food composition. The betaine and the coconut oil may be present in an amount of from about 1% to about 5%, from about 1.5% to about 4.5%, or from about 1.8% to about 3.8%, based on the dry weight of the pet food composition. The carnitine and the coconut oil may be present in an amount of from about 1% to about 5%, from about 1.5% to about 4%, or from about 1.5% to about 3.5%, based on the dry weight of the pet food composition. The betaine, carnitine and coconut oil may be present in an amount of from about 1% to about 5%, from about 1.5% to about 4%, or from about 1.8% to about 3.8%, based on the dry weight of the pet food composition.

### EXAMPLES

The examples and other implementations described herein are exemplary and not intended to be limiting the invention which is defined in the claims.

### Example 1

Thirty-two dogs were fed for 90 days with one of four treatments. The first treatment group was given food containing 2.3% of a source of medium chain triglycerides (e.g., coconut oil) (Treatment 1). The second treatment group was given food containing 300 ppm carnitine and 2.3% of a source of medium chain triglycerides (e.g., coconut oil) (Treatment 2). The third treatment group was given food containing 5,000 ppm betaine and 2.3% of a source of medium chain triglycerides (e.g., coconut oil) (Treatment 3). The fourth treatment group was given food containing 5,000 ppm betaine, 300 ppm carnitine and 2.3% of a source of medium chain triglycerides (e.g., coconut oil) (Treatment 4). Treatment 4 is according to the invention. Treatments 1-3 are reference treatments. Initial and end of study values were measured for body composition using a dual energy x-ray absorption technology. The results are shown in Table 1 (below).

**Table 1: Mass change during the feeding period.**

| | Treatment 1 | Treatment 2 | Treatment 3 | Treatment 4 |
|---|---|---|---|---|
| Lean (grams) | 106 | 24 | 150 | 269 |
| Fat (grams) | -261 | -183 | -144 | -415 |
| Weight (grams) | -159 | -166 | 7 | -154 |

While each treatment group showed an increase in the lean mass of the animals, Treatment 4 (which contained an exemplary combination of the present invention) surprisingly showed a synergistic positive effect. Significantly, the increase in lean mass was observed at the same time as the overall weight was reduced for Treatments groups 1, 2, and 4; while Treatment group 3 (which was fed betaine and coconut oil) showed a weight gain.

The individual effects due to the addition of carnitine or betaine within the food resulted in a decrease in body fat. Surprisingly and unexpectedly, an enhanced synergistic effect was seen in the reduction of fat when these ingredients were combined within the pet food (see, Treatment 4).

## Claims

1. A pet food composition comprising:
betaine,
carnitine, and
a source of medium chain triglycerides;
wherein the weight ratio of betaine to carnitine is from 13:1 to 20:1, and the weight ratio of betaine to the source of medium chain triglycerides is from 1:3 to 1:7.

2. The pet food composition according to claim 1, wherein the source of medium chain triglycerides is selected from: coconut oil, palm kernel oil, palm oil, a structured lipid of medium chain triglycerides, and a combination of two or more thereof.

3. The pet food composition according to claim 1 or claim 2, wherein the source of medium chain triglycerides provides at least one medium chain triglyceride selected from: hexanoic acid, octanoic acid, decanoic acid, and dodecanoic acid.

4. The pet food composition according to any foregoing claim, wherein the source of medium chain triglycerides comprises coconut oil.

5. The pet food composition according to any foregoing claim, wherein the betaine is present in an amount of 0.03% to 1%, 0.2% to 0.95%, or 0.3% to 0.85%, based on the dry weight of the pet food composition.

6. The pet food composition according to any foregoing claim, wherein the carnitine is present in an amount of 0.01% to 0.06%, 0.015% to 0.055%, or 0.015% to 0.05%, based on the dry weight of the pet food composition.

7. The pet food composition according to any foregoing claim, wherein the source of medium chain triglycerides is present in an amount of 1% to 4%, 1.5% to 3.5%, or 1.5% to 3%, based on the dry weight of the pet food composition.

8. The pet food composition according to any foregoing claim, wherein the weight ratio of betaine to carnitine is from 14:1 to 19:1, or from 15:1 to 18:1, or 16.66:1.

9. The pet food composition according to any foregoing claim, wherein the weight ratio of betaine to the source of medium chain triglycerides is from 1:3.3 to 1:6, or from 1:3.6 to 1:5.2, or 1:4.6.

10. The pet food composition according to any foregoing claim, wherein the weight ratio of carnitine to the source of medium chain triglycerides is from 1:60 to 1:90, from 1:65 to 1:85, or from 1:70 to 1:80, or 1:76.66.

11. The pet food composition according to any foregoing claim, wherein the total amount of betaine and carnitine is from 0.3% to 1%, from 0.4% to 0.8%, or from 0.4% to 0.6%, based on the dry weight of the pet food composition.

12. The pet food composition according to any foregoing claim, wherein the total amount of betaine and the source of medium chain triglycerides is from 1% to 5%, from 1.5% to 4.5%, or from 1.8% to 3.8%, based on the dry weight of the pet food composition.

13. The pet food composition according to any foregoing claim, wherein the total amount of carnitine and the source of medium chain triglycerides is from 1% to 5%, from 1.5% to 4%, or from 1.5% to 3.5%, based on the dry weight of the pet food composition.

14. The pet food composition according to any foregoing claim, wherein the total amount of betaine, carnitine and the source of medium chain triglycerides are present in an amount of about 1% to 5%, from 1.5% to 4%, or from 1.8% to 3.8%, based on the dry weight of the pet food composition.

## Patentansprüche

1. Haustierfutterzusammensetzung, umfassend:
Betain,
Carnitin und
eine Quelle für mittelkettige Triglyceride;
wobei das Gewichtsverhältnis von Betain zu Carnitin 13:1 bis 20:1 beträgt und das Gewichtsverhältnis von Betain zu der Quelle für mittelkettige Triglyceride 1:3 bis 1:7 beträgt.

2. Haustierfutterzusammensetzung nach Anspruch 1, wobei die Quelle für mittelkettige Triglyceride ausgewählt ist unter: Kokosnussöl, Palmkernöl, Palmöl, einem strukturierten Lipid aus mittelkettigen Triglyceriden und einer Kombination von zwei oder mehreren davon.

3. Haustierfutterzusammensetzung nach Anspruch 1 oder Anspruch 2, wobei die Quelle für mittelkettige Triglyceride mindestens ein mittelkettiges Triglycerid bereitstellt, das ausgewählt ist unter: Hexansäure, Octansäure, Decansäure und Dodecansäure.

4. Haustierfutterzusammensetzung nach einem der vorstehenden Ansprüche, wobei die Quelle für mittelkettige Triglyceride Kokosnussöl umfasst.

5. Haustierfutterzusammensetzung nach einem der vorstehenden Ansprüche, wobei das Betain in einer Menge von 0,03% bis 1%, 0,2% bis 0,95% oder 0,3% bis 0,85%, bezogen auf das Trockengewicht der Haustierfutterzusammensetzung, vorhanden ist.

6. Haustierfutterzusammensetzung nach einem der vorstehenden Ansprüche, wobei das Carnitin in einer Menge von 0,01% bis 0,06%, 0,015% bis 0,055% oder 0,015% bis 0,05%, bezogen auf das Trockengewicht der Haustierfutterzusammensetzung, vorhanden ist.

7. Haustierfutterzusammensetzung nach einem der vorstehenden Ansprüche, wobei die Quelle für mittelkettige Triglyceride in einer Menge von 1% bis 4%, 1,5% bis 3,5% oder 1,5% bis 3%, bezogen auf das Trockengewicht der Haustierfutterzusammensetzung, vorhanden ist.

8. Tierfutterzusammensetzung nach einem der vorstehenden Ansprüche, wobei das Gewichtsverhältnis von Betain zu Carnitin von 14:1 bis 19:1 oder von 15:1 bis 18:1 oder 16,66:1 beträgt.

9. Haustierfutterzusammensetzung nach einem der vorstehenden Ansprüche, wobei das Gewichtsverhältnis von Betain zu der Quelle von mittelkettigen Triglyceriden von 1:3,3 bis 1:6 oder von 1:3,6 bis 1:5,2 oder 1:4,6 beträgt.

10. Haustierfutterzusammensetzung nach einem der vorstehenden Ansprüche, wobei das Gewichtsverhältnis von Carnitin zu der Quelle von mittelkettigen Triglyceriden von 1:60 bis 1:90, von 1:65 bis 1:85 oder von 1:70 bis 1:80 oder 1:76,66 beträgt.

11. Haustierfutterzusammensetzung nach einem der vorstehenden Ansprüche, wobei die Gesamtmenge an Betain und Carnitin von 0,3% bis 1%, von 0,4% bis 0,8% oder von 0,4% bis 0,6% beträgt, bezogen auf das Trockengewicht der Haustierfutterzusammensetzung.

12. Haustierfutterzusammensetzung nach einem der vorstehenden Ansprüche, wobei die Gesamtmenge an Betain und der Quelle für mittelkettige Triglyceride von 1% bis 5%, von 1,5% bis 4,5% oder von 1,8% bis 3,8% beträgt, bezogen auf das Trockengewicht der Haustierfutterzusammensetzung.

13. Haustierfutterzusammensetzung nach einem der vorstehenden Ansprüche, wobei die Gesamtmenge an Carnitin und der Quelle für mittelkettige Triglyceride von 1% bis 5%, von 1,5% bis 4% oder von 1,5% bis 3,5% beträgt, bezogen auf das Trockengewicht der Haustierfutterzusammensetzung.

14. Haustierfutterzusammensetzung nach einem der vorstehenden Ansprüche, wobei die Gesamtmenge an Betain, Carnitin und der Quelle für mittelkettige Triglyceride in einer Menge von etwa 1% bis 5%, von 1,5% bis 4% oder von 1,8% bis 3,8%, bezogen auf das Trockengewicht der Haustierfutterzusammensetzung, vorhanden ist.

## Revendications

1. Composition alimentaire pour animaux de compagnie comprenant :
de la bétaïne,
de la carnitine et
une source de triglycérides à chaîne moyenne ;
dans laquelle le rapport pondéral de la bétaïne à la carnitine est de 13:1 à 20:1, et le rapport pondéral de la bétaïne à la source de triglycérides à chaîne moyenne est de 1:3 à 1:7.

2. Composition alimentaire pour animaux de compagnie selon la revendication 1, dans laquelle la source de triglycérides à chaîne moyenne est choisie parmi : l'huile de coco, l'huile de palmiste, l'huile de palme, un lipide structuré de triglycérides à chaîne moyenne, et une combinaison de deux ou plus de ceux-ci.

3. Composition alimentaire pour animaux de compagnie selon la revendication 1 ou 2, dans laquelle la source de triglycérides à chaîne moyenne fournit au moins un triglycéride à chaîne moyenne choisi parmi : l'acide hexanoïque, l'acide octanoïque, l'acide décanoïque et l'acide dodécanoïque.

4. Composition alimentaire pour animaux de compagnie selon l'une quelconque des revendications précédentes, dans laquelle la source de triglycérides à chaîne moyenne comprend de l'huile de coco.

5. Composition alimentaire pour animaux de compagnie selon l'une quelconque des revendications précédentes, dans laquelle la bétaïne est présente en une quantité de 0,03 % à 1 %, de 0,2 % à 0,95 % ou de 0,3 % à 0,85 %, par rapport au poids sec de la composition alimentaire pour animaux de compagnie.

6. Composition alimentaire pour animaux de compagnie selon l'une quelconque des revendications précédentes, dans laquelle la carnitine est présente en une quantité de 0,01 % à 0,06 %, de 0,015 % à 0,055 %, ou de 0,015 % à 0,05 %, par rapport au poids sec de la composition alimentaire pour animaux de compagnie.

7. Composition alimentaire pour animaux de compagnie selon l'une quelconque des revendications précédentes, dans laquelle la source de triglycérides à chaîne moyenne est présente en une quantité de 1 % à 4 %, de 1,5 % à 3,5 %, ou de 1,5 % à 3 %, par rapport au poids sec de la composition alimentaire pour animaux de compagnie.

8. Composition alimentaire pour animaux de compagnie selon l'une quelconque des revendications précédentes, dans laquelle le rapport pondéral de la bétaïne à la carnitine est de 14:1 à 19:1, ou de 15:1 à 18:1, ou de 16,66:1.

9. Composition alimentaire pour animaux de compagnie selon l'une quelconque des revendications précédentes, dans laquelle le rapport pondéral de la bétaïne à la source de triglycérides à chaîne moyenne est de 1:3,3 à 1:6, ou de 1:3,6 à 1:5,2, ou de 1:4,6.

10. Composition alimentaire pour animaux de compagnie selon l'une quelconque des revendications précédentes, dans laquelle le rapport pondéral de la carnitine à la source de triglycérides à chaîne moyenne est de 1:60 et 1:90, de 1:65 à 1:85, ou de 1:70 à 1:80, ou de 1:76,66.

11. Composition alimentaire pour animaux de compagnie selon l'une quelconque des revendications précédentes, dans laquelle la quantité totale de bétaïne et de carnitine est de 0,3 % à 1 %, de 0,4 % à 0,8 %, ou de 0,4 % à 0,6 %, par rapport au poids sec de la composition alimentaire pour animaux de compagnie.

12. Composition alimentaire pour animaux de compagnie selon l'une quelconque des revendications précédentes, dans laquelle la quantité totale de bétaïne et de la source de triglycérides à chaîne moyenne est de 1 % à 5 %, de 1,5 % à 4,5 %, ou de 1,8 % et 3,8 %, par rapport au poids sec de la composition alimentaire pour animaux de compagnie.

13. Composition alimentaire pour animaux de compagnie selon l'une quelconque des revendications précédentes, dans laquelle la quantité totale de carnitine et de la source de triglycérides à chaîne moyenne est de 1 % à 5 %, de 1,5 % à 4 %, ou de 1,5 % à 3,5 %, par rapport au poids sec de la composition alimentaire pour animaux de compagnie.

14. Composition alimentaire pour animaux de compagnie selon l'une quelconque des revendications précédentes, dans laquelle la quantité totale de bétaïne, de carnitine et de la source de triglycérides à chaîne moyenne sont présentes en une quantité d'environ 1 % à 5 %, de 1,5 % à 4 %, ou de 1,8 % à 3,8 %, par rapport au poids sec de la composition alimentaire pour animaux de compagnie.
